(19) Europäisches Patentamt
European Patent Office
Office européen des brevets

(11) EP 0 897 715 A2

# (12) EUROPÄISCHE PATENTANMELDUNG

(43) Veröffentlichungstag:
**24.02.1999 Patentblatt 1999/08**

(51) Int. Cl.$^6$: **A61K 7/42**

(21) Anmeldenummer: **98114607.9**

(22) Anmeldetag: **04.08.1998**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **19.08.1997 DE 19735901**

(71) Anmelder:
**Beiersdorf Aktiengesellschaft**
**20245 Hamburg (DE)**

(72) Erfinder:
**Gers-Barlag, Heinrich, Dr.**
**25495 Kummerfeld (DE)**

## (54) Kosmetische oder dermatologische Lichtschutzmittel, welche als Festkörper vorliegende UV-Filtersubstanzen und polymere UV-Filtersubstanzen auf Siliconbasis enthalten

(57) Lichtschutzwirksame Wirkstoffkombinationen aus unter Normalbedingungen als Festkörper vorliegenden organischen UV-Filterkomponenten und oligomeren oder polymeran UV-Flltersubstenzen mit periodisch sich wiederholenden Si-O- Gruppen.

EP 0 897 715 A2

**Beschreibung**

Die vorliegende Erfindung betrifft Lichtschutzformulierungen, insbesondere kosmetische und dermatologische Lichtschutzmittel.

Die schädigende Wirkung des ultravioletten Teils der Sonnenstrahlung auf die Haut ist allgemein bekannt. Während Strahlen mit einer Wellenlänge, die kleiner als 290 nm ist (der sogenannte UVC-Bereich), von der Ozonschicht in der Erdatmosphäre absorbiert werden, verursachen Strahlen im Bereich zwischen 290 nm und 320 nm, dem sogenannten UVB-Bereich, ein Erythem, einen einfachen Sonnenbrand oder sogar mehr oder weniger starke Verbrennungen.

Als ein Maximum der Erythemwirksamkeit des Sonnenlichtes wird der engere Bereich um 308 nm angegeben.

Zum Schutz gegen UVB-Strahlung sind zahlreiche Verbindungen bekannt, bei denen es sich um Derivate des 3-Benzylidencamphers, der 4-Aminobenzoesäure, der Zimtsäure, der Salicylsäure, des Benzophenons sowie auch des 2-Phenylbenzimidazols handelt.

Auch für den Bereich zwischen etwa 320 nm und etwa 400 nm, den sogenannten UVA-Bereich, ist es wichtig, Filtersubsubstanzen zur Verfügung zu haben, da auch dessen Strahlen Schäden hervorrufen können. So ist erwiesen, daß UVA-Strahlung zu einer Schädigung der elastischen und kollagenen Fasern des Bindegewebes führt, was die Haut vorzeitig altem läßt, und daß sie als Ursache zahlreicher phototoxischer und photoallergischer Reaktionen zu sehen ist. Der schädigende Einfluß der UVB-Strahlung kann durch UVA-Strahlung verstärkt werden.

Zum Schutz gegen die Strahlen des UVA-Bereichs werden daher gewisse Derivate des Dibenzoylmethans verwendet, deren Photostabilität (Int. J. Cosm. Science 10, 53 (1988)) nicht in ausreichendem Maße gegeben ist.

Die UV-Strahlung kann aber auch zu photochemischen Reaktionen führen, wobei dann die photochemischen Reaktionsprodukte in den Hautmetabolismus eingreifen. Vorwiegend handelt es sich bei solchen photochemischen Reaktionsprodukten um radikalische Verbindungen, beispielsweise Hydroxyradikale. Auch undefinierte radikalische Photoprodukte, welche in der Haut selbst entstehen, können aufgrund ihrer hohen Reaktivität unkontrollierte Folgereaktionen an den Tag legen. Aber auch Singulettsauerstoff, ein nichtradikalischer angeregter Zustand des Sauerstoffmoleküls kann bei UV-Bestrahlung auftreten, ebenso kurzlebige Epoxide und viele Andere. Singulettsauerstoff beispielsweise zeichnet sich gegenüber dem normalerweise vorliegenden Triplettsauerstoff (radikalischer Grundzustand) durch gesteigerte Reaktivität aus. Allerdings existieren auch angeregte, reaktive (radikalische) Triplettzustände des Sauerstoffmoleküls.

Um diesen Reaktionen vorzubeugen, können den kosmetischen bzw. dermatologischen Formulierungen zusätzlich Antioxidantien und/oder Radikalfänger einverleibt werden.

Im allgemeinen ist das Lichtabsorptionsverhalten von Lichtschutzfiltersubstanzen sehr gut bekannt und dokumentiert, zumal in den meisten Industrieländern Positivlisten für den Einsatz solcher Substanzen existieren, welche recht strenge Maßstäbe an die Dokumentation anlegen. Für die Dosierung der Substanzen in den fertigen Formulierungen können die Extinktionswerte allenfalls eine Orientierungshilfe bieten, denn durch Wechselwirkungen mit Inhaltsstoffen der Haut oder der Hautoberfläche selbst können Unwägbarkeiten auftreten. Ferner ist in der Regel schwierig vorab abzuschätzen, wie gleichmäßig und in welcher Schichtdicke die Filtersubstanz in und auf der Hornschicht der Haut verteilt ist.

Der Lichtschutzfaktor (LSF, oft auch, dem englischen Sprachgebrauch angepaßt, SPF genannt) gibt an, um wieviel länger die mit dem Lichtschutzmittel geschützte Haut bestrahlt werden kann, bis die gleiche Erythemreaktion auftritt wie bei der ungeschützten Haut (also zehnmal solang gegenüber ungeschützter Haut bei LSF = 10).

Jedenfalls erwartet der Verbraucher zum einen - nicht zuletzt wegen der ins Licht der Öffentlichkeit gerückten Diskussion über das sogenannte „Ozonloch" zum einen zuverlässige Angaben des Herstellers zum Lichtschutzfaktor, zum anderen geht eine Tendenz des Verbrauchers zu höheren und hohen Lichtschutzfaktoren.

Kosmetische oder dermatologische UV-Filtersubstanzen liegen in Lichtschutzformulierungen üblicherweise in gelöster Form vor. Gemäß Formulierungen des Standes der Technik war es erheblich erschwert, solche Substanzen als Festkörper derart homogen verteilt in den entsprechenden Formulierungen einzusetzen, daß, ohne einen erheblichen Wirksamkeitsverlust einigermaßen befriedigende Produkte erhältlich waren.

Da Lichtschutzfiltersubstanzen in der Regel kostspielig sind, und da manche Lichtschutzfiltersubstanzen zudem schwierig in höheren Konzentrationen in kosmetische oder dermatologische Zubereitungen einzuarbeiten sind, war es Aufgabe der Erfindung, auf einfache und preiswerte Weise zu Zubereitungen zu gelangen, welche bei ungewöhnlich niedrigen Konzentrationen an herkömmlichen Lichtschutzfiltersubstanzen dennoch akzeptable oder sogar hohe LSF-Werte erreichen.

Bekannte und vorteilhafte Lichtschutzfiltersubstanzen sind Dibenzoylmethanderivate, beispielsweise 5-Isopropyldibenzoylmethan (CAS-Nr. 63250-25-9), welches sich durch die Struktur

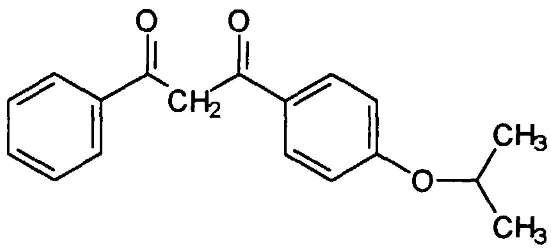

auszeichnet und von Merck unter der Marke Eusolex® 8020 verkauft wird, sowie das 4-(tert.-Butyl)-4'-methoxydibenzoylmethan (CAS-Nr. 70356-09-1), welches sich durch die Struktur

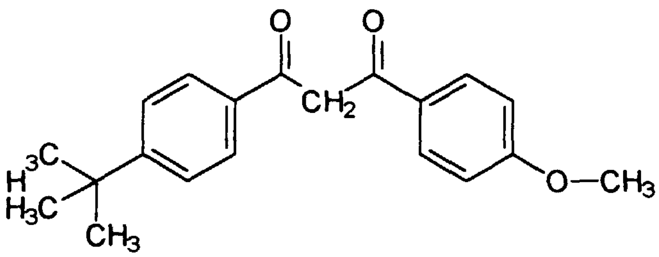

auszeichnet, und von Givaudan unter der Marke Parsol® 1789 verkauft wird. Gerade in Kombination mit anderen als Festkörper vorliegenden Substanzen ist ihre Einsatzkonzentration jedoch begrenzt. Es bereitet daher gewisse formulierungstechnische Schwierigkeiten, höhere Lichtschutzfaktoren zu erzielen.

Eine weitere vorteilhafte Lichtschutzfiltersubstanz ist der 4-Methylbenzylidencampher, welcher sich durch die Struktur

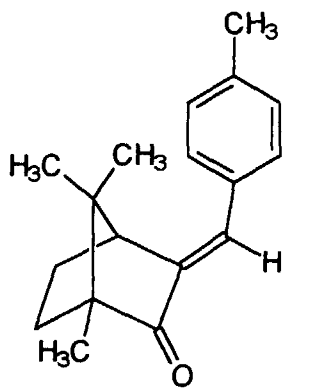

auszeichnet und von Merck unter der Marke Eusolex® 6300 verkauft wird. 4-Methyl-benzylidencampher ist eine äußerst vorteilhafte, unter Normalbedingungen als Festkörper vorliegende Lichtschutzfiltersubstanz und zeichnet sich an sich durch gute UV-Filtereigenschaften aus. Gerade in Kombination mit anderen als Festkörper vorliegenden Substanzen ist jedoch auch ihre Einsatzkonzentration jedoch begrenzt. Es bereitet daher auch hier gewisse formulierungstechnische Schwierigkeiten, höhere Lichtschutzfaktoren zu erzielen.

Auch andere Benzylidencampherderivate sind vorteilhafte Lichtschutzfiltersubstanzen, z.B. der Benzylidencampher, welcher sich durch die Struktur

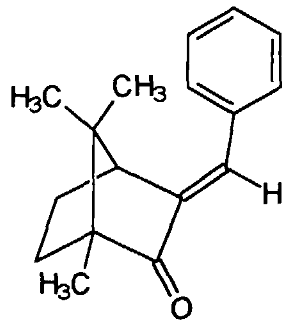

auszeichnet und von der Gesellschaft Induchem unter der Marke Unisol® S22 verkauft wird. Gerade in Kombination mit anderen als Festkörper vorliegenden Substanzen ist jedoch auch ihre Einsatzkonzentration jedoch begrenzt. Es bereitet daher auch hier gewisse formulierungstechnische Schwierigkeiten, höhere Lichtschutzfaktoren zu erzielen.

Ein weiterer vorteilhafter UV-Filter ist der 4,4',4"-(1,3,5-Triazin-2,4,6-triyltriimino)-tris-benzoësäure-tris(2-ethylhexylester), synonym: 2,4,6-Tris-[anilino-(p-carbo-2'-ethyl-1'-hexyloxy)]-1,3,5-triazin.

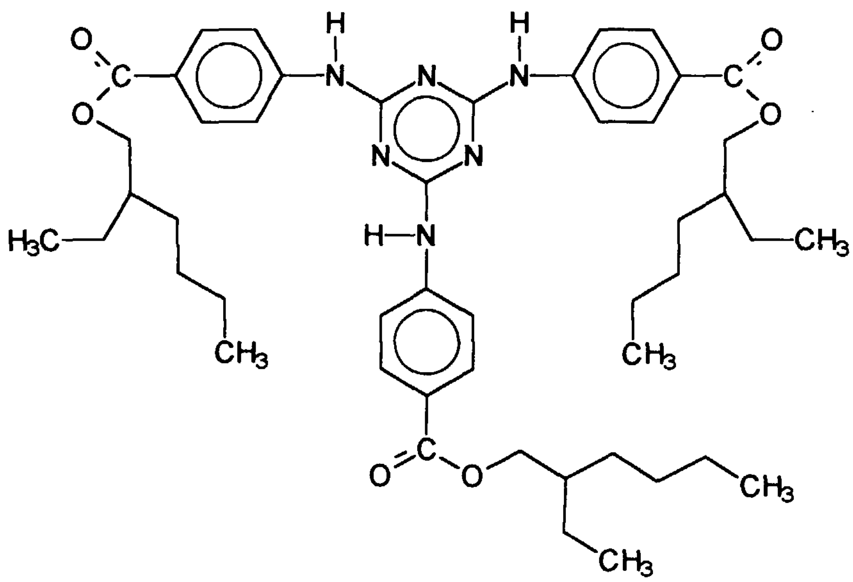

Diese UVB-Filtersubstanz wird von der BASF Aktiengesellschaft unter der Warenbezeichnung UVINUL® T 150 vertrieben und zeichnet sich durch gute UV-Absorptionseigenschaften aus. Zwischenzeitlich wurden von verschiedenen Autoren andere UV-Filtersubstanzen beschrieben, welche das Strukturmotiv

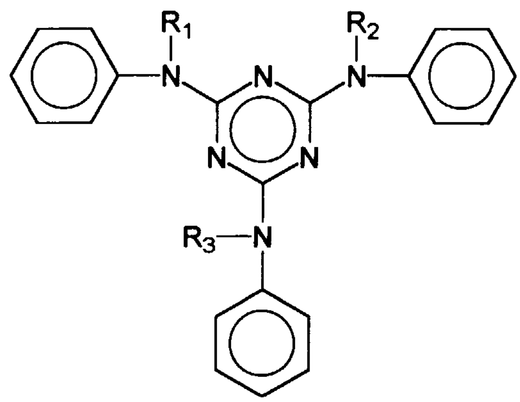

aufweisen.

Der Hauptnachteil des 4,4',4"-(1,3,5-Triazin-2,4,6-triyltriimino)-tris-benzoësäure-tris(2-ethylhexylesters) ist seine schlechte Löslichkeit in Lipiden. Bekannte Lösungsmittel für diesen UVB-Filter können maximal ca. 15 Gew.-% dieses Filters lösen, entsprechend etwa 1 - 1,5 Gew.-% gelöster, und damit aktiver, UV-Filtersubstanz. Es bereitet daher auch hier gewisse formulierungstechnische Schwierigkeiten, höhere Lichtschutzfaktoren zu erzielen.

Auch andere als Festkörper vorliegende UV-Filtersubstanzen, deren Einarbeitung in kosmetische oder dermatologische Lichtschutzformulierungen zumindest gewisse Probleme aufweist, sind bekannt. So werden in der EP-A-570 838 s-Triazinderivate beschrieben, deren chemische Struktur durch die generische Formel

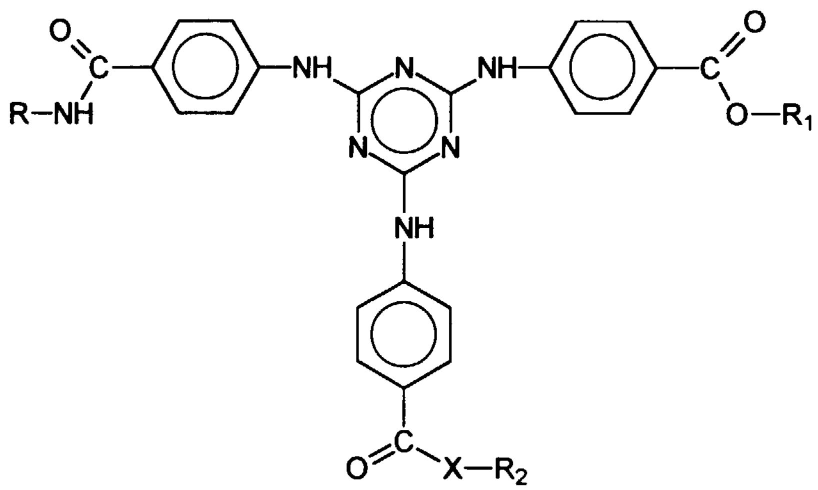

wiedergegeben wird, wobei

- R einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, darstellt,
- X ein Sauerstoffatom oder eine NH-Gruppe darstellt,
- $R_1$ einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, oder ein Wasserstoffatom, ein Alkalimetallatom, eine Ammoniumgruppe oder eine Gruppe der Formel

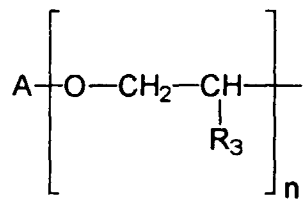

bedeutet, in welcher

A einen verzweigten oder unverzweigten $C_1$-$6_{18}$-Alkylrest, einen $C_5$-$C_{12}$-Cycloalkyl- oder Arylrest darstellt, gegebenenfalls substituiert mit einer oder mehreren $C_1$-$C_4$- Alkylgruppen,

$R_3$ ein Wasserstoffatom oder eine Methylgruppe darstellt,

n eine Zahl von 1 bis 10 darstellt,

$R_2$ einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, darstellt, wenn X die NH-Gruppe darstellt, und einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, oder ein Wasserstoffatom, ein Alkalimetallatom, eine Ammoniumgruppe oder eine Gruppe der Formel

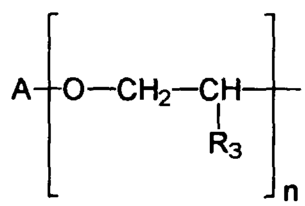

bedeutet, in welcher

A einen verzweigten oder unverzweigten $C_1$-$C_{18}$-Alkylrest, einen $C_5$-$C_{12}$-Cycloalkyl- oder Arylrest darstellt, gegebenenfalls substituiert mit einer oder mehreren $C_1$-$C_4$- Alkylgruppen,

$R_3$ ein Wasserstoffatom oder eine Methylgruppe darstellt,

n eine Zahl von 1 bis 10 darstellt,

wenn X ein Sauerstoffatom darstellt.

Selbst wenn grundsätzlich ein gewisser UV-Schutz bei gegebener begrenzter Löslichkeit, (und damit nach herkömmlichen Maßstäben: schlechter Einarbeitbarkeit in eine kosmetische oder dermatologische Zubereitung) erreicht werden kann, kann ein anderes Problem auftreten, die Rekristallisation. Diese tritt gerade bei schlecht löslichen Substanzen vergleichsweise schnell ein, sei es durch Temperaturschwankungen oder andere Einflüsse hervorgerufen. Unkontrollierte Rekristallisation eines wesentlichen Zubereitungsbestandteiles wie eines UV-Filters hat aber äußerst nachteilige Einwirkungen auf die Eigenschaften der gegebenen Zubereitung und, nicht zuletzt, auf den angestrebten Lichtschutz.

Auch andere UV-Filtersubstanzen, deren Einarbeitung in kosmetische oder dermatologische Lichtschutzformulierungen zumindest gewisse Probleme aufweist, sind bekannt. So werden in der EP-A-775 698 Bis-Resorcinyltriazinderivate beschrieben, deren chemische Struktur durch die generische Formel

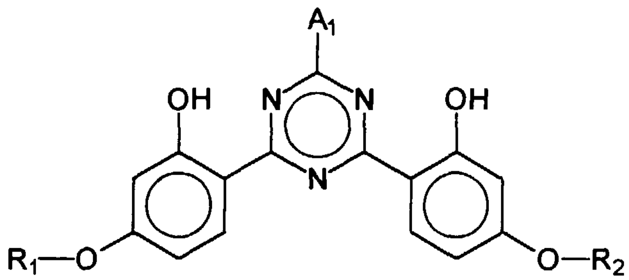

wiedergegeben wird, wobei $R_1$ , $R_2$ und $A_1$ verschiedenste organische Reste repräsentieren.

Vorteilhafte Bis-Resorcinyltriazinderivate sind beispielsweise folgende Verbindungen:

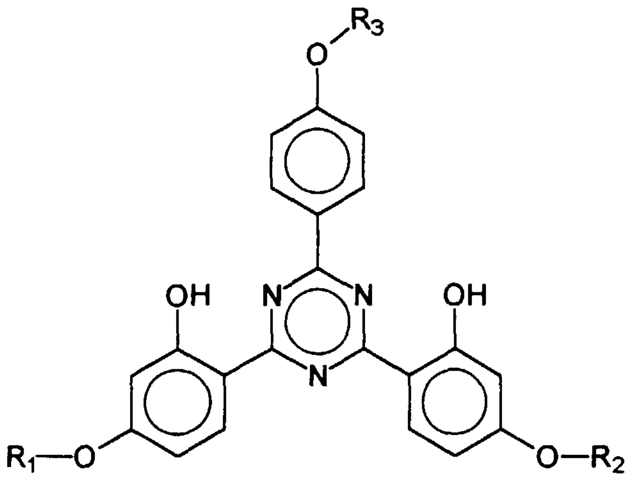

wobei $R_3$ ein Wasserstoffatom oder eine verzweigte oder unverzweigte Alkylgruppe mit 1 bis 10 Kohlenstoffatomen darstellt, insbesondere das 2,4-Bis-{[4-(2-Ethyl-hexyloxy)-2-hydroxy]-phenyl}-6-(4-methoxyphenyl)-1,3,5-triazin, welches durch folgende Struktur gekennzeichnet ist:

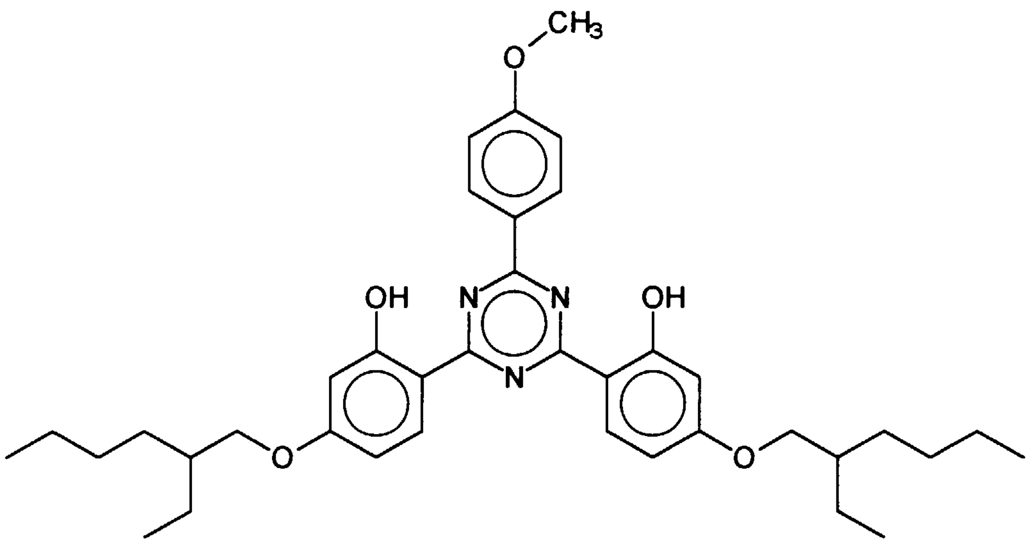

Ferner vorteilhaft ist das 2,4-Bis-{[4-(3-sulfonato)-2-hydroxy-propyloxy)-2-hydroxy]-phenyl}-6-(4-methoxphenyl)-1,3,5-triazin Natriumsalz, welches durch folgende Struktur gekennzeichnet ist:

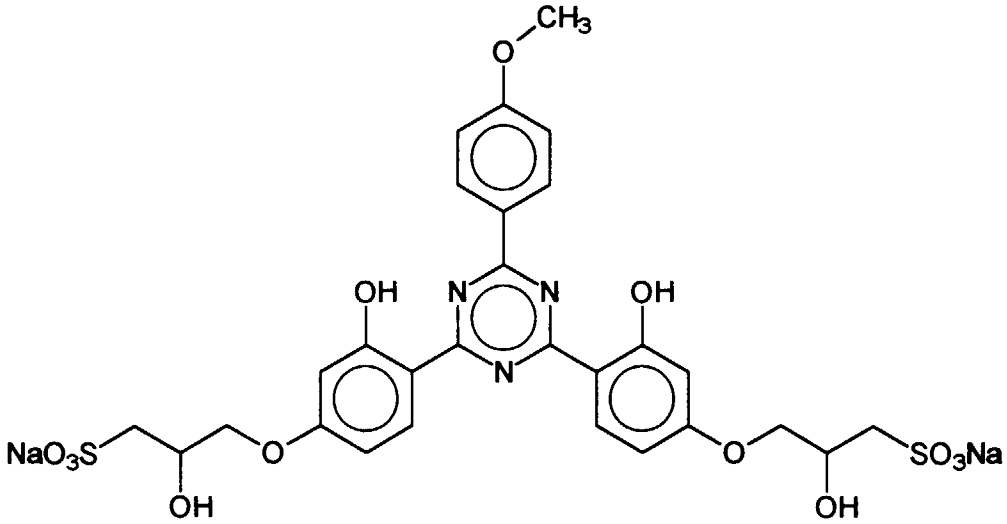

Ferner vorteilhaft ist das 2,4-Bis-{[4-(3-(2-Propyloxy)-2-hydroxy-propyloxy)-2-hydroxy]-phenyl}-6-(4-methoxyphenyl)-1,3,5-triazin, welches durch folgende Struktur gekennzeichnet ist:

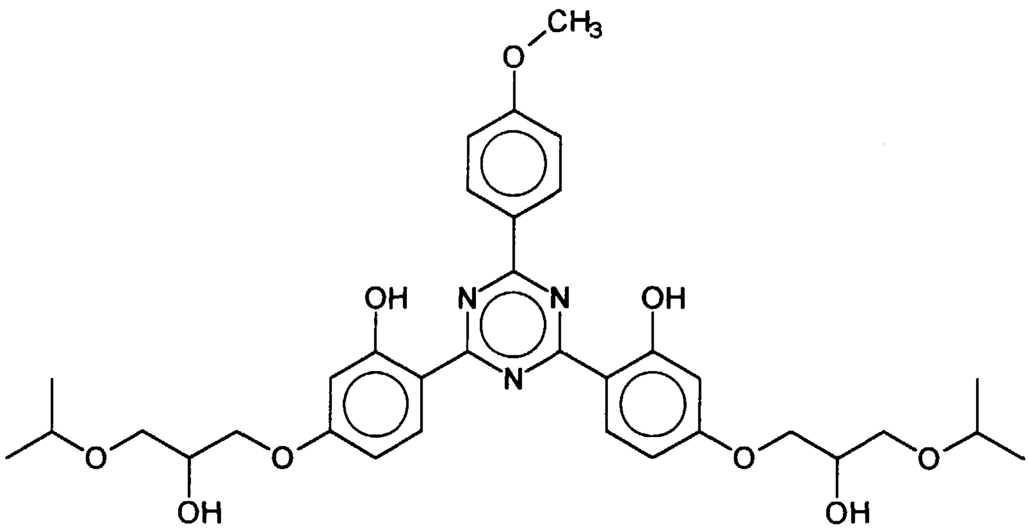

Ferner vorteilhaft ist das 2,4-Bis-{[4-(2-ethyl-hexyloxy)-2-hydroxy]-phenyl}-6-[4-(2-methoxyethyl-carboxyl)-phenylamino]-1,3,5-triazin, welches durch folgende Struktur gekennzeichnet ist:

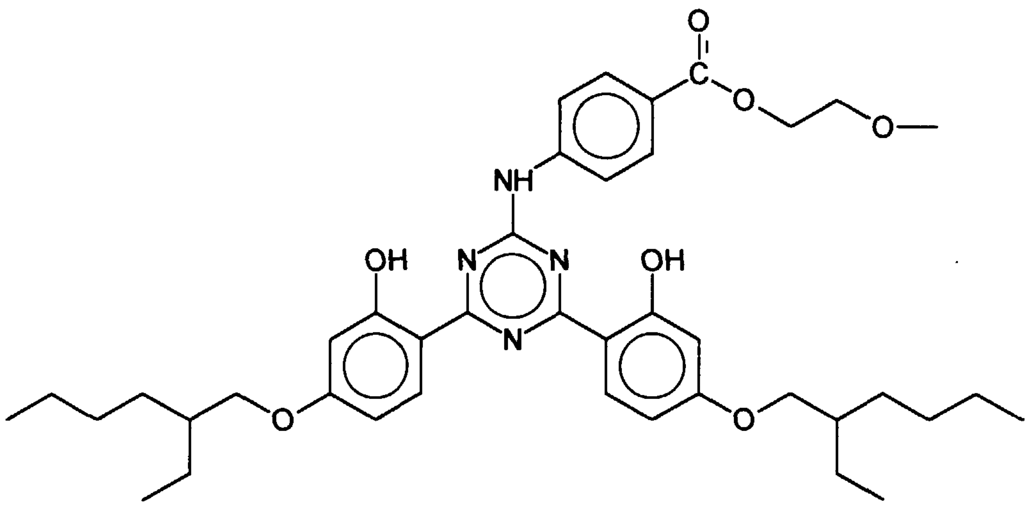

Ferner vorteilhaft ist das 2,4-Bis-{[4-(3-(2-propyloxy)-2-hydroxy-propyloxy)-2-hydroxy]-phenyl}-6-[4-(2-ethyl-carboxyl)-phenylamino]-1,3,5-triazin, welches durch folgende Struktur gekennzeichnet ist:

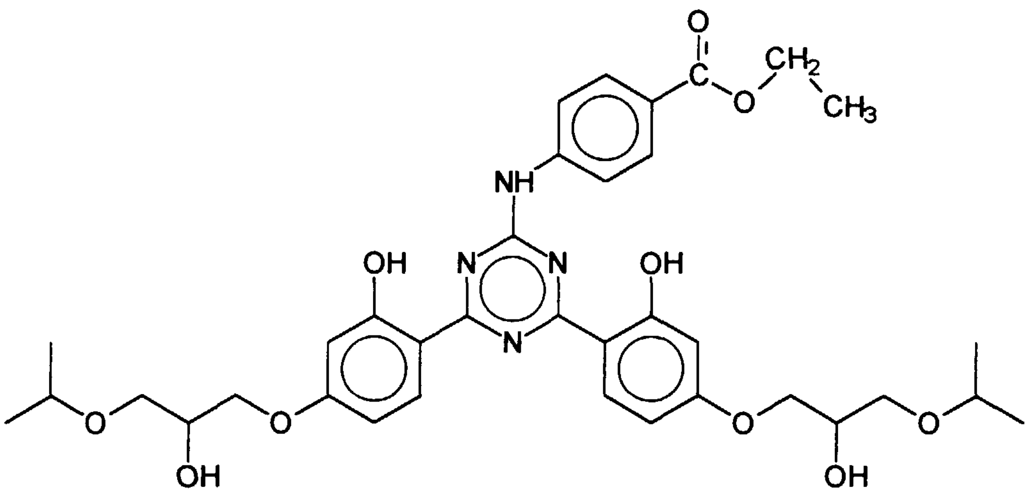

Ferner vorteilhaft ist das 2,4-Bis-{[4-(2-ethyl-hexyloxy)-2-hydroxy]-phenyl}-6-(1-methyl-pyrrol-2-yl)-1,3,5-triazin, welches durch folgende Struktur gekennzeichnet ist:

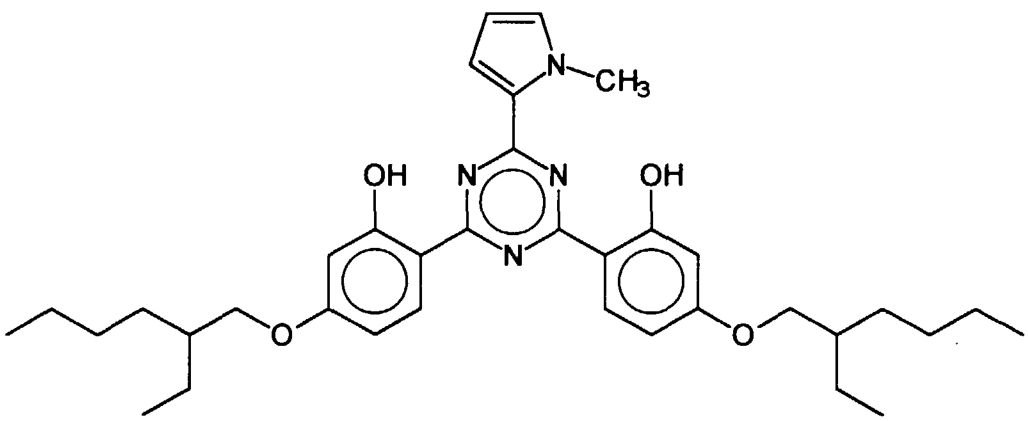

Ferner vorteilhaft ist das 2,4-Bis-{[4-tris(trimethylsiloxy-silylpropyloxy)-2-hydroxy]-phenyl}-6-(4-methoxyphenyl)-1,3,5-triazin, welches durch folgende Struktur gekennzeichnet ist:

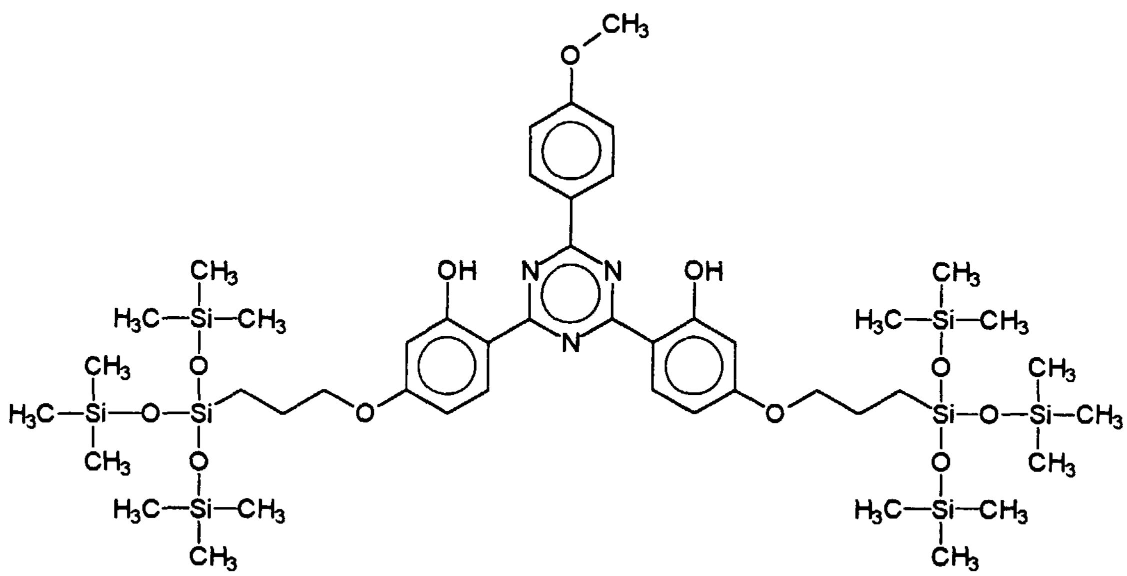

Ferner vorteilhaft ist das 2,4-Bis-{[4-(2"-methylpropenyloxy)-2-hydroxy]-phenyl}-6-(4-methoxyphenyl)-1,3,5-triazin, welches durch folgende Struktur gekennzeichnet ist:

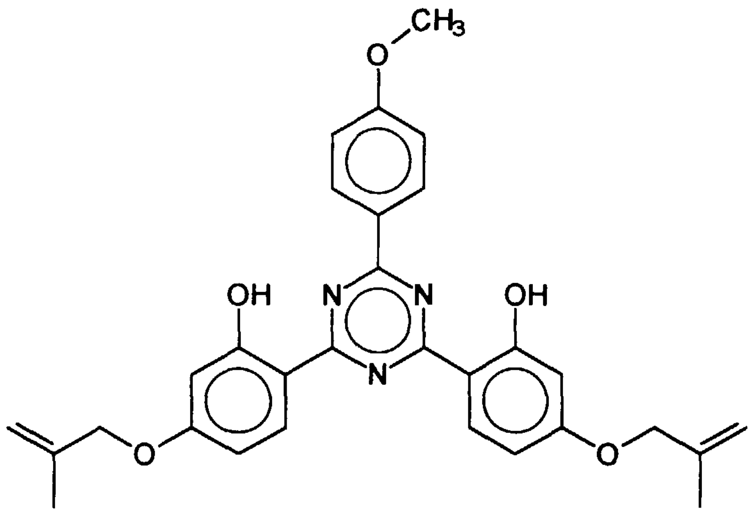

Ferner vorteilhaft ist das 2,4-Bis-{[4-(1′,1′,1′,3′,5′,5′,5′-Heptamethylsiloxy-2"-methyl-propyloxy)-2-hydroxy]-phenyl}-6-(4-methoxyphenyl)-1,3,5-triazin, welches durch folgende Struktur gekennzeichnet ist:

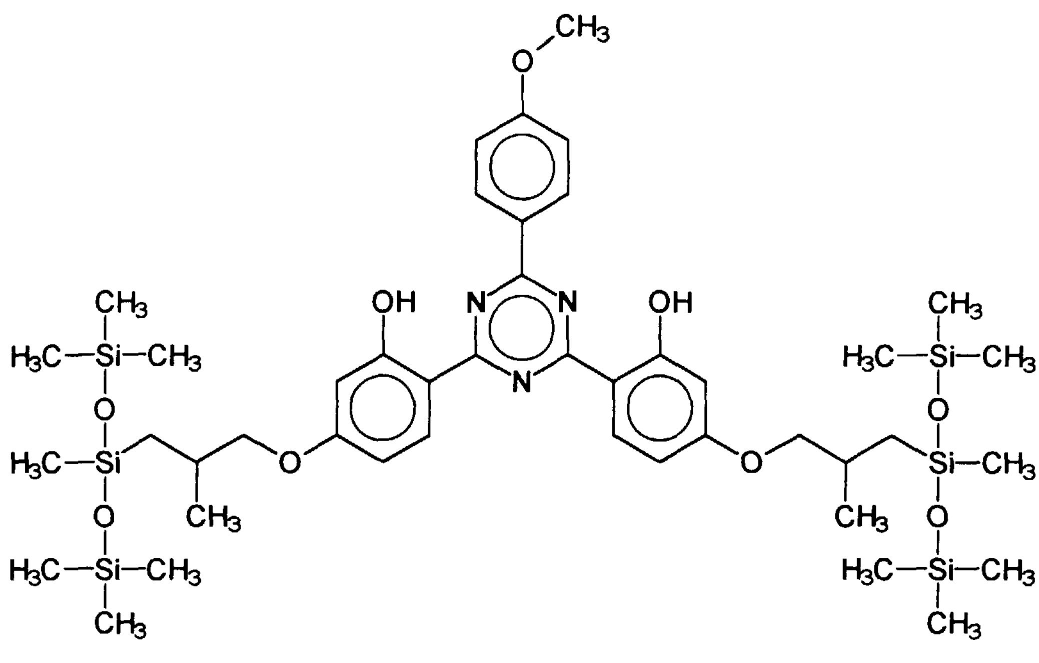

Selbst wenn grundsätzlich ein gewisser UV-Schutz bei gegebener begrenzter Löslichkeit, (und damit nach herkömmlichen Maßstäben: schlechter Einarbeitbarkeit in eine kosmetische oder dermatologische Zubereitung) mit Bis-Resorcinyltriazinderivaten erreicht werden kann, kann ein anderes Problem auftreten, die Rekristallisation. Diese tritt gerade bei schlecht löslichen Substanzen vergleichsweise schnell ein, sei es durch Temperaturschwankungen oder andere Einflüsse hervorgerufen. Unkontrollierte Rekristallisation eines wesentlichen Zubereitungsbestandteiles wie eines UV-Filters hat aber äußerst nachteilige Einwirkungen auf die Eigenschaften der gegebenen Zubereitung und, nicht zuletzt, auf den angestrebten Lichtschutz.

Noch ein weiterer vorteilhafter UV-Filter ist das 2,2′-Methylen-bis-(6-(2H-benzotriazol-2-yl)-4-(1,1,3,3-tetramethylbutyl)-phenol), welches durch die chemische Strukturformel

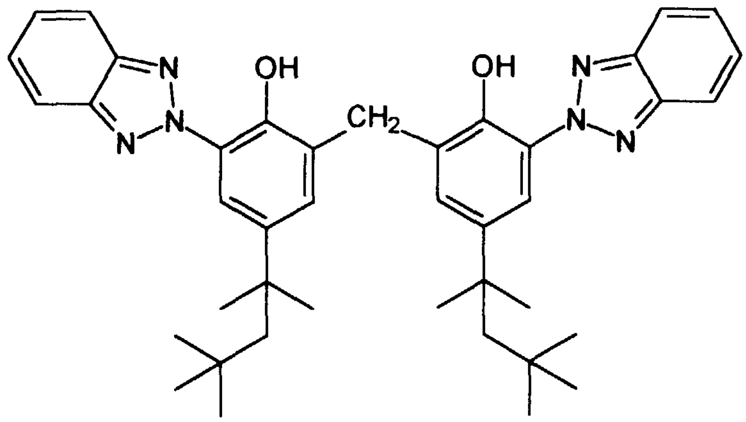

gekennzeichnet ist. Dennoch ergeben sich auch bei dieser Substanz gewisse, formulierungstechnisch bedingte Nachteile bei Einsatz hoher Mengen, denn diese Substanz ist schwerlöslich. Es ist unter Verwendung dieser Substanz daher schwierig, hohe Lichtschutzfaktoren zu erreichen.

Auch andere als Festkörper vorliegende - oftmals schwerlösliche - UV-Filtersubstanzen sind bekannt, beispielsweise 2-Phenylbenzimidazol-5-sulfonsäure bzw. ihre Salze, insbesondere das Natrium-, das Kalium und das TEA-Salz, beispielsweise erhältlich unter der Bezeichnung Eusolex® 232 der Merck AG, welches sich durch folgende Strukturformel auszeichnet:

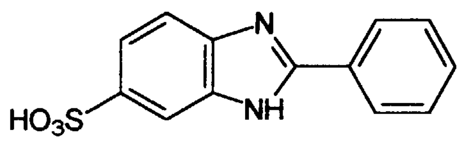

Die teilweise vorstehend genannten Verbindungen, welche als Lichtschutzmittel für kosmetische und dermatologische Lichtschutzformulierungen eingesetzt werden, zeichnen sich, wie gesagt, an sich durch gute Lichtschutzwirkung aus. Sie haben jedoch den Nachteil, daß es bisweilen schwierig ist, sie in befriedigender Weise solchen Formulierungen einzuverleiben.

In den Schriften EP-A-392 883, WO 92/20690 und WO 93/04665 werden vorteilhafte UV-Filtersubstanzen beschrieben, welche sich dadurch auszeichnen, daß chromophore Gruppen mit guten UV-Absorptionseigenschaften an ein Grundgerüst gebunden sind, welches als Organosiloxan charakterisiert werden kann.

Es war indes überraschend, daß lichtschutzwirksame Wirkstoffkombinationen aus unter Normalbedingungen als Festkörper vorliegenden organischen UV-Filterkomponenten und oligomeren oder polymeren UV-Filtersubstanzen mit periodisch sich wiederholenden Si-O- Gruppen bzw. kosmetische oder pharmazeutische Zubereitungen mit einem Gehalt an solchen Wirkstoffkombinationen die Nachteile des Standes der Technik beseitigen.

Erfindungsgemäß können die oligomeren oder polymeren UV-Filtersubstanzen mit periodisch sich wiederholenden Si-O- Gruppen vorteilhaft aus der Gruppe der UV-Filtersubstanzen gewählt werden, die in den Schriften EP-A-392 883, WO 92/20690 und WO 93/04665 UV-Filtersubstanzen beschrieben werden.

Grob schematisch können diese oligomeren oder polymeren UV-Filtersubstanzen, die im folgenden der Einfachheit halber auch „Polymerfilter" genannt werden sollen, durch folgende allgemeine Strukturen beschrieben werden:

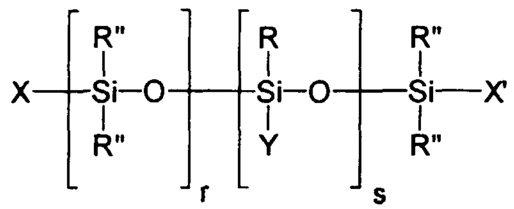

(Strukturschema I)

bzw.

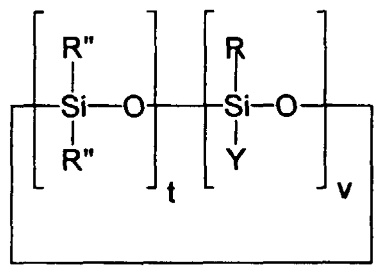

(Strukturschema II)

Dabei repräsentieren die Reste R und R" allgemeine organische Reste, die Reste X, X' und repräsentieren entweder ebenfalls organische Reste wie R und R" oder aber Reste wie Y, was weiter unten erläutert wird. r kann Werte von 0 - 100 annehmen, s kann Werte von 0 - 20 annehmen, wobei mindestens einer der Rest e X, X' einen Rest Y bedeutet, wenn s = 0. t kann Werte von 0 - 10 annehmen, v kann Werte von 1 - 10 annehmen, wobei die Summe aus v und t größer oder gleich 3 ist.

Y stellt die für die UV-Absorption verantwortlichen Chromophore dar. Insbesondere repräsentieren die Reste R und R" Alkylreste mit 1 - 10 Kohlenstoffatomen bzw. Phenylreste. Die Chromophorreste Y werden insbesondere vorteilhaft gewählt aus der Gruppe

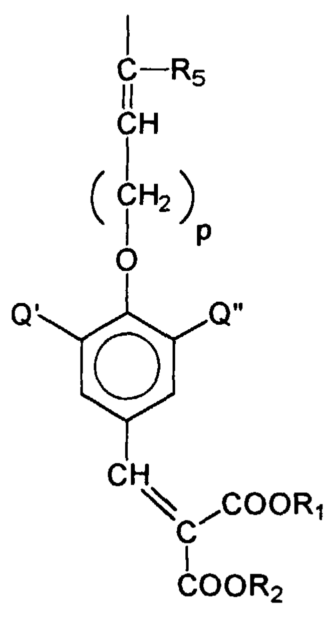

und/oder

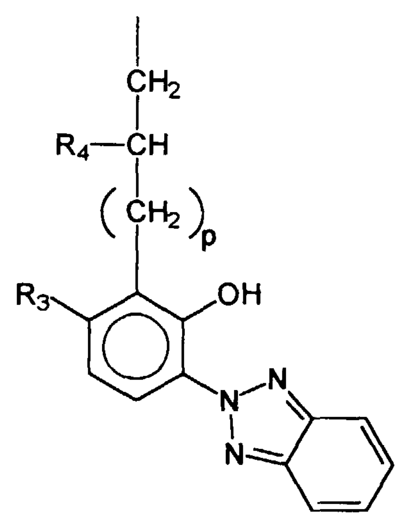

,

wobei $R_1$, $R_2$, unabhängig voneinander verzweigte oder unverzweigte Alkylgruppen von 1 - 10 Kohlenstoffatomen darstellen, und wobei $R_3$, $R_4$, $R_5$ unabhängig voneinander Wasserstoffatome bzw. verzweigte oder unverzweigte Alkylgruppen von 1 - 10 Kohlenstoffatomen darstellen und p eine Zahl von 1 - 10 darstellt. Q' und/oder Q" können

unabhängig voneinander darstellen: H, Hydroxygruppen, verzweigte oder unverzweigte Alkylgruppen von 1 - 10 Kohlenstoffatomen, verzweigte oder unverzweigte Alkoxygruppen mit 1 - 10 Kohlenstoffatomen.

Ganz besonders vorteilhaft wird Y gewählt aus der Gruppe

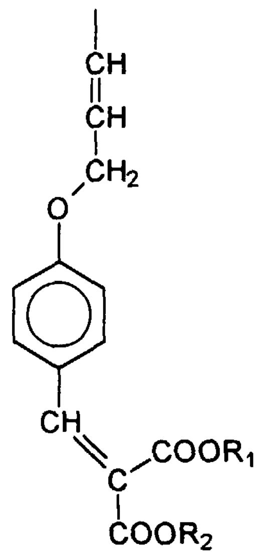
,
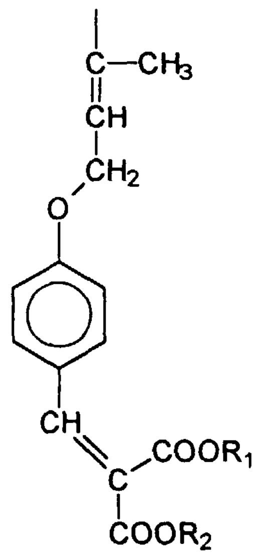
,
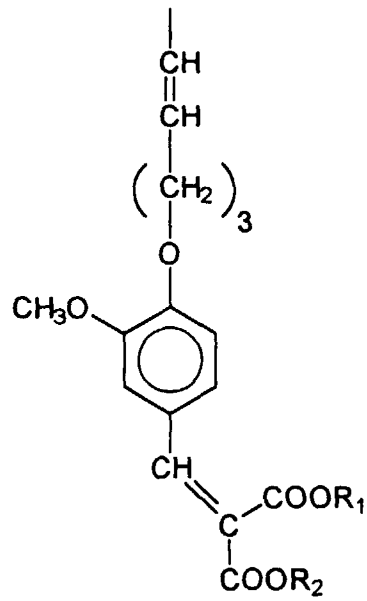
,

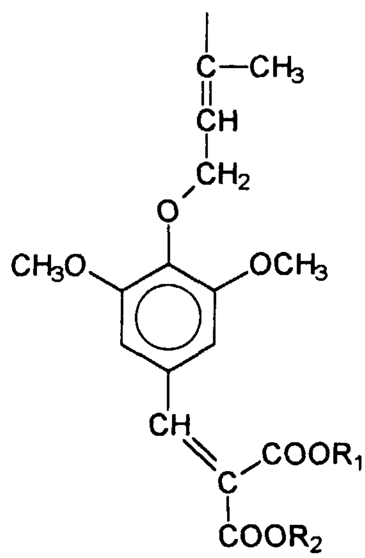
,

wobei $R_1$ und $R_2$ unabhängig voneinander Methyl oder Ethyl darstellen.

Bevorzugter erfindungsgemäß verwendeter Polymerfilter sind Substanzen, in welchen Y die Gruppe

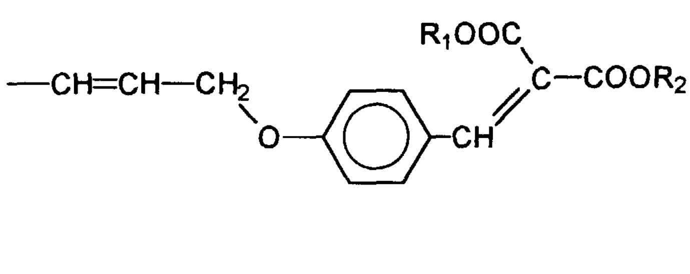

darstellt, wobei R bzw. R" Methylgruppen darstellen, und in in welchen $R_1$ und/oder $R_2$ Ethyl- und/oder Methylgruppen darstellen und bei welcher das stöchiometrische Verhältnis von Si-O- Einheiten, welche Chromophorgruppen Y tragen, zu Si-O- Einheiten, welche keine Chromophorgruppen Y tragen, im Bereich von 1 : 20 bis 1 : 10, insbesondere etwa 1 : 15 liegt.

Besonders vorteilhafte Ausführungsformen der vorliegenden Erfindung werden auch erhalten, wenn als Y gewählt wird der Rest

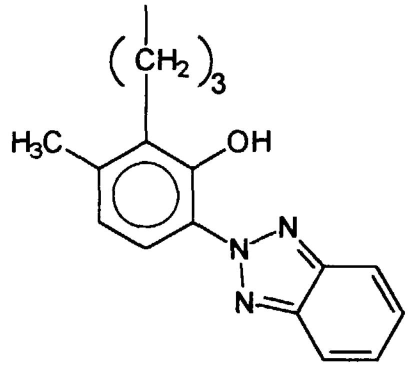

.

Es war überraschend und für den Fachmann nicht vorauszusehen, daß die Verwendung von erfindungsgemäß verwendeten Polymerfiltern zur Erhöhung des Lichtschutzfaktors kosmetischer oder dermatologischer Zubereitungen, welche mindestens eine unter Normalbedingungen als Festkörper vorliegende UV-Filtersubstanz enthalten, führen würde.

Insbesondere war erstaunlich, daß die Polymerfilter im Sinne der vorliegenden Erfindung zu einer Steigerung des Lichtschutzfaktors führen würden, wenn wenigstens einer der UV-Filter in den Lichtschutzzubereitungen gemäß der vorliegenden Erfindung aus der Gruppe der Triazinderivate gewählt wird, insbesondere gewählt aus der Gruppe

- 4,4',4"-(1,3,5-Triazin-2,4,6-triyltriimino)-tris-benzoesäure-tris(2-ethylhexylester)
- 2,4-Bis-{[4-(2-Ethyl-hexyloxy)-2-hydroxy]-phenyl}-6-(4-methoxyphenyl)-1,3,5-triazin,
- 2,4-Bis-{[4-(3-sulfonato)-2-hydroxy-propyloxy)-2-hydroxy]-phenyl}-6-(4-methoxy-phenyl)-1,3,5-triazin Natriumsalz,
- 2,4-Bis-{[4-(3-(2-Propyloxy)-2-hydroxy-propyloxy)-2-hydroxy]-phenyl}-6-(4-methoxyphenyl)-1,3,5-triazin,
- 2,4-Bis-{[4-(2-ethyl-hexyloxy)-2-hydroxy]-phenyl}-6-[4-(2-methoxyethyl-carboxyl)-phenylamino]-1,3,5-triazin,
- 2,4-Bis-{[4-(3-(2-propyloxy-2-hydroxy-propyloxy)-2-hydroxy]-phenyl}-6-[4-(2-ethyl-carboxyl)-phenylamino]-1,3,5-triazin,
- 2,4-Bis-{[4-2-ethyl-hexyloxy)-2-hydroxy]-phenyl}-6-(1-methyl-pyrrol-2-yl)-1,3,5-triazin,
- 2,4-Bis-{[4-tris(trimethylsiloxy-silylpropyloxy)-2-hydroxy]-phenyl}-6-(4-methoxyphenyl)-1,3,5-triazin,
- 2,4-Bis-{[4-(2"-methylpropenyloxy)-2-hydroxy]-phenyl}-6-(4-methoxyphenyl)-1,3,5-triazin,
- 2,4-Bis-{[4-(1',1',1',3',5',5',5'-Heptamethylsiloxy-2"-methyl-propyloxy)-2-hydroxy]-phenyl}-6-(4-methoxyphenyl)-1,3,5-triazin.

Ferner war erstaunlich, daß die Polymerfilter im Sinne der vorliegenden Erfindung zu einer Steigerung des Lichtschutzfaktors führen würden, wenn wenigstens einer der UV-Filter in den Lichtschutzzubereitungen gemäß der vorliegenden Erfindung das 2,2'-Methylen-bis-(6-(2H-benzotriazol-2-yl)-4-(1,1,3,3-tetramethylbutyl)-phenol) ist.

Ferner war erstaunlich, daß die Polymerfilter im Sinne der vorliegenden Erfindung zu einer besonders homogenen Verteilung der unter Normalbedingungen als Festkörper vorliegenden UV-Filtersubstanzen führen würden, wenn wenigstens eine dieser als Festkörper vorliegenden UV-Filtersubstanzen in den Lichtschutzzubereitungen gemäß der vorliegenden Erfindung aus der Gruppe der Triazinderivate gewählt wird, insbesondere gewählt aus der Gruppe

- 4,4',4"-(1,3,5-Triazin-2,4,6-triyltriimino)-tris-benzoesäure-tris(2-ethylhexylester)
- 2,4-Bis-{[4-(2-Ethyl-hexyloxy)-2-hydroxy]-phenyl}-6-(4-methoxyphenyl)-1,3,5-triazin,
- 2,4-Bis-{[4-(3-sulfonato)-2-hydroxy-propyloxy)-2-hydroxy]-phenyl}-6-(4-methoxyphenyl)-1,3,5-triazin Natriumsalz,
- 2,4-Bis-{[4-(3-(2-Propyloxy-2-hydroxy-propyloxy)-2-hydroxy]-phenyl}-6-(4-methoxyphenyl)-1,3,5-triazin,
- 2,4-Bis-{[4-(2-ethyl-hexyloxy)-2-hydroxy]-phenyl}-6-[4-(2-methoxyethyl-carboxyl)-phenylamino]-1,3,5-triazin,
- 2,4-Bis-{[4-(3-(2-propyloxy)-2-hydroxy-propyloxy)-2-hydroxy]-phenyl}-6-[4-(2-ethyl-carboxyl)-phenylamino]-1,3,5-triazin,

- 2,4-Bis-{[4-(2-ethyl-hexyloxy)-2-hydroxy]-phenyl}-6-(1-methyl-pyrrol-2-yl)-1,3,5-triazin,
- 2,4-Bis-{[4-tris(trimethylsiloxy-silylpropyloxy)-2-hydroxy]-phenyl}-6-(4-methoxyphenyl)-1,3,5-triazin,
- 2,4-Bis-{[4-(2"-methylpropenyloxy)-2-hydroxy]-phenyl}-6-(4-methoxyphenyl)-1,3,5-triazin,
- 2,4-Bis-{[4-(1',1',1',3',5',5',5'-Heptamethylsiloxy-2"-methyl-propyloxy)-2-hydroxy]-phenyl}-6-(4-methoxyphenyl)-1,3,5-triazin.

Weiterhin war erstaunlich, daß die Polymerfilter im Sinne der vorliegenden Erfindung zu einer besonders homogenen Verteilung der unter Normalbedingungen als Festkörper vorliegenden UV-Filtersubstanzen führen würden, wenn wenigstens einer der UV-Filter in den Lichtschutzzubereitungen gemäß der vorliegenden Erfindung das 2,2'-Methylen-bis-(6-(2H-benzotriazol-2-yl)-4-(1,1,3,3-tetramethylbutyl)-phenol) ist.

Erfindungsgemäß ist daher ferner die Verwendung von oligomeren oder polymeren UV-Filtersubstanzen mit periodisch sich wiederholenden Si-O- Gruppen zur Erzielung, Erhöhung bzw. Verbesserung der Dispergierbarkeit von unter Normalbedingungen als Festkörper vorliegenden UV-Filtersubstanzen in kosmetischen oder pharmazeutischen Zubereitungen.

Die Gesamtmenge an erfindungsgemäß verwendeten Polymerfiltern in den erfindungsgemäßen kosmetischen oder dermatologischen Zubereitungen wird vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an erfindungsgemäß verwendeten, unter Normalbedingungen als Festkörper vorliegenden UV-Filtersubstanzen in den erfindungsgemäßen kosmetischen oder dermatologischen Zubereitungen wird vorteilhaft aus dem Bereich von 0,1 - 10,0 Gew.-%, bevorzugt 0,5 - 6,0 Gew.-% gewählt, bezogen auf das Gesamtgewicht der Zubereitungen.

Grundsätzlich ist zwar möglich und vorteilhaft, die erfindungsgemäß verwendeten, unter Normalbedingungen als Festkörper vorliegenden UV-Filtersubstanzen zu jedem beliebigen Zeitpunkt dem den Emulsionen zugrundeliegenden Gemisch zuzugeben. Es wird allerdings erfindungsgemäß bevorzugt, die erfindungsgemäß verwendeten, unter Normalbedingungen als Festkörper vorliegenden UV-Filtersubstanzen vor einem eventuell erwünschten Emulgierungsvorgang in der Ölphase, gegebenenfalls unter Erwärmen, zu dispergieren und weitere übliche Verarbeitungsschritte nachzuschalten.

Es ist bevorzugt, die erfindungsgemäß verwendeten unter Normalbedingungen als Festkörper vorliegenden UV-Filtersubstanzen unter Erwärmen in der Ölphase, insbesondere vorteilhaft in einer unpolaren Ölkomponente bzw. einem Gemisch aus unpolaren Ölkomponenten, zu dispergieren.

Es ist dabei bevorzugt, zunächst die Löslichkeit der erfindungsgemäß verwendeten unter Normalbedingungen als Festkörper vorliegenden UV-Filtersubstanzen in der Gesamtheit der Ölkomponenten zu bestimmen, wobei es von Vorteil ist, solche Ölkomponenten auszuwählen, in denen die erfindungsgemäß verwendeten unter Normalbedingungen als Festkörper vorliegenden UV-Filtersubstanzen zu höchstens 5 Gew.-% löslich sind. Diese Vorauswahl zu treffen, übersteigt nicht das allgemeine Wissen des Fachmannes.

Erfindungsgemäße kosmetische und dermatologische Zubereitungen enthalten vorteilhaft, wenngleich nicht zwingend, anorganische Pigmente auf Basis von Metalloxiden und/oder anderen in Wasser schwerlöslichen oder unlöslichen Metallverbindungen, insbesondere der Oxide des Titans ($TiO_2$), Zinks ($ZnO$), Eisens (z.B. $Fe_2O_3$, Zirkoniums ($ZrO_2$), Siliciums ($SiO_2$), Mangans (z.B. $MnO$), Aluminiums ($Al_2O_3$), Cers (z.B. $Ce_2O_3$), Mischoxiden der entsprechenden Metalle sowie Abmischungen aus solchen Oxiden. Besonders bevorzugt handelt es sich um Pigmente auf der Basis von $TiO_2$.

Solche anorganischen Pigmente liegen erfindungsgemäß vorteilhaft in hydrophober Form vor, d.h., daß sie oberflächlich wasserabweisend behandelt sind. Diese Oberflächenbehandlung kann darin bestehen, daß die Pigmente nach an sich bekannten Verfahren mit einer dünnen hydrophoben Schicht versehen werden.

Eines solcher Verfahren besteht beispielsweise darin, daß die hydrophobe Oberflächenschicht nach einer Reaktion gemäß

$$n\ TiO_2 + m\ (RO)_3Si\text{-}R' \rightarrow n\ TiO_2\ (\text{oberfl.})$$

erzeugt wird. n und m sind dabei nach Belieben einzusetzende stöchiometrische Parameter, R und R' die gewünschten organischen Reste. Beispielsweise in Analogie zu DE-OS 33 14 742 dargestellte hydrophobisierte Pigmente sind von Vorteil.

Vorteilhafte $TiO_2$-Pigmente sind beispielsweise unter den Handelsbezeichnungen T 805 von der Firma Degussa erhältlich.

Zubereitungen gemäß der Erfindung können auch röntgenamporphe Oxidpigmente enthalten. Röntgenamorphe Oxidpigmente sind Metalloxide oder Halbmetalloxide, welche bei Röntgenbeugungsexperimenten keine oder keine erkenntliche Kristallstruktur erkennen lassen. Oftmals sind solche Pigmente erhältlich durch Flammenreaktion, beispielsweise dadurch, daß ein Metall- oder Halbmetallhalogenid mit Wasserstoff und Luft (oder reinem Sauerstoff) in

einer Flamme umgesetzt wird.

Bekannte und in der kosmetischen oder dermatologischen Galenik oftmals verwendete röntgenamorphe Oxidpigmente sind die Siliciumoxide des Typs Aerosil® (CAS-Nr. 7631-86-9. Aerosile®, erhältlich von der Gesellschaft DEGUSSA, zeichnen sich durch geringe Partikelgröße (z.B. zwischen 5 und 40 nm) aus, wobei die Partikel als kugelförmige Teilchen sehr einheitlicher Abmessung anzusehen sind. Makroskopisch sind Aerosil® als lockere, weiße Pulver erkenntlich. Im Sinne der vorliegenden Erfindung sind röntgenamorphe Siliciumdioxidpigmente besonders vorteilhaft, und unter diesen gerade solche des Aerosil®-Typs bevorzugt.

Vorteilhafte Aerosil®-Typen sind beispielsweise Aerosil® OX50, Aerosil® 130, Aerosil® 150, Aerosil® 200, Aerosil® 300, Aerosil® 380, Aerosil® MOX 80, Aerosil® MOX 170, Aerosil® COK 84, Aerosil® R 202, Aerosil® R 805, Aerosil® R 812, Aerosil® R 972, Aerosil® R 974, Aerosil® R976.

Erfindungsgemäß enthalten kosmetische oder dermatologische Lichtschutzzubereitungen 0,1 bis 20 Gew.-%, vorteilhaft 0,5 bis 10 Gew.-%, ganz besonders bevorzugt 1 bis 5 Gew.-% röntgenamorphe Oxidpigmente.

Die Gesamtmenge an anorganischen Pigmenten, insbesondere hydrophoben anorganischen Mikropigmenten in den fertigen kosmetischen oder dermatologischen Zubereitungen wird vorteilhaft aus dem Bereich von 0,1 - 30 Gew.-%, bevorzugt 0,1 - 10,0, insbesondere 0,5 - 6,0 Gew.-% gewählt, bezogen auf das Gesamtgewicht der Zubereitungen.

Die erfindungsgemäßen kosmetischen und/oder dermatologischen Lichtschutzformulierungen können wie üblich zusammengesetzt sein und dem kosmetischen und/oder dermatologischen Lichtschutz, ferner zur Behandlung, der Pflege und der Reinigung der Haut und/oder der Haare und als Schminkprodukt in der dekorativen Kosmetik dienen.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Besonders bevorzugt sind solche kosmetischen und dermatologischen Zubereitungen, die in der Form eines Sonnenschutzmittels vorliegen. Vorteilhaft können diese zusätzlich mindestens einen weiteren UVA-Filter und/oder mindestens einen weiteren UVB-Filter und/oder mindestens ein anorganisches Pigment, bevorzugt ein anorganisches Mikropigment, enthalten.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. $\alpha$-Carotin, $\beta$-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl, Oleyl-, $\gamma$-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homooysteinsulfoximin, Buthioninsulfone, Penta-, Hexa-, Heptathioninsulfoximin) in sehr geringen verträglichen Dosierungen (z.B. pmol bis $\mu$mol/kg), ferner (Metall)-Chelatoren (z.B. $\alpha$-Hydroxyfettsäuren, Palmitinsäure, Phytinsäure, Lactoferrin), $\alpha$-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. $\gamma$-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, $\alpha$-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, $ZnSO_4$) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Die Lipidphase kann vorteilhaft gewählt werden aus folgender Substanzgruppe:

- Mineralöle, Mineralwachse
- Öle, wie Triglyceride der Caprin- oder der Caprylsäure, ferner natürliche Öle wie z.B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylherylisostearat, Octyldodecanol, Isotridecylisononanoat, Isoeicosan, 2-Ethylhexylcocoat, $C_{12-15}$-Alkylbenzoat, Capryl-Caprinsäure-triglycerid, Dicaprylylether.

Besonders vorteilhaft sind Mischungen aus $C_{12-15}$-Alkylbenzoat und 2-Ethylherylisostearat, Mischungen aus $C_{12-15}$-Alkylbenzoat und Isotridecylisononanoat sowie Mischungen aus $C_{12-15}$-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wäßrige Phase der erfindungsgemäßen Zubereitungen enthält gegebenenfalls vorteilhaft

- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder - monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Erfindungsgemäß wird der LSF durch die Verwendung von Polymerfiltern in kosmetischen oder dermatologischen Zubereitungen, enthaltend übliche UVA-Filter und/oder UVB-Filter in der Lipidphase und/oder in der wäßrigen Phase erheblich gesteigert.

Insbesondere wird der LSF durch die Verwendung von Polymerfiltern in kosmetischen oder dermatologischen Zubereitungen, enthaltend Substanzen, die UV-Strahlung im UVB-Bereich absorbieren, wobei die Gesamtmenge solcher Filtersubstanzen z.B. 0,1 Gew.-% bis 30 Gew.-%, vorzugsweise 0,5 bis 20 Gew.-%, insbesondere 3 bis 15 Gew.-% beträgt, bezogen auf das Gesamtgewicht der Zubereitungen, erheblich gesteigert.

Solche UVB-Filter können öllöslich oder wasserlöslich sein. Vorteilhafte öllösliche UVB-Filtersubstanzen sind z.B.:

- 4-Aminobenzoesäure-Derivate, vorzugsweise 4-(Dimethylamino)-benzoesäure(2-ethylhexyl)ester, 4-(Dimethylamino)benzoesäureamylester;
- 2,4,6-Trianilino-(p-carbo-2'-ethyl-1'-hexyloxy)-1,3,5-triazin;
- Ester der Benzalmalonsäure, vorzugsweise 4-Methoxybenzalmalonsäuredi(2-ethylhexyl)ester;
- Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;

  Vorteilhafte wasserlösliche UVB-Filtersubstanzen sind z.B.:
- Sulfonsäure-Derivate des 3-Benzylidencamphers, wie z.B. 4-(2-Oxo-3-bornylidenmethyl)benzolsulfonsäure,2-Methyl-5-(2-oxo-3-bornylidenmethyl)sulfonsäure und deren Salze.

Die Liste der genannten UVB-Filter, die zusätzlich im Sinne der vorliegenden Erfindung eingesetzt werden können, soll selbstverständlich nicht limitierend sein.

Insbesondere wird der LSF durch die Verwendung von Polymerfiltern in kosmetischen oder dermatologischen Zubereitungen, enthaltend Substanzen, die UV-Strahlung im UVA-Bereich absorbieren, wobei die Gesamtmenge solcher Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen, erheblich gesteigert.

Die Gesamtmenge an wasserlöslichen UV-Filtersubstanzen in den fertigen kosmetischen oder dermatologischen Zubereitungen wird vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 2-Phenylbenzimidazol-5-sulfonsäure bzw. deren Salzen in den fertigen kosmetischen oder dermatologischen Zubereitungen wird, falls die Gegenwart dieser Substanz erwünscht ist, vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 2-Hydroxy-4-methoxybenzophenon-5-sulfonsäurebzw. deren Salzen in den fertigen kosmetischen oder dermatologischen Zubereitungen wird, falls die Gegenwart dieser Substanz erwünscht ist, vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 4-(2-Oxo-3-bornylidenmethyl)benzolsulfonsäure bzw. deren Salzen in den fertigen kosmetischen oder dermatologischen Zubereitungen wird, falls die Gegenwart dieser Substanz erwünscht ist, vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 2-Methyl-5-(2-oxo-3-bornylidenmethyl)benzolsulfonsäur bzw. deren Salzen in den fertigen kosmetischen oder dermatologischen Zubereitungen wird, falls die Gegenwart dieser Substanz erwünscht ist, vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an Benzol-1,4-di(2-oxo-3-bornylidenmethyl-10-Sulfonsäurebzw. deren Salzen in den fertigen kosmetischen oder dermatologischen Zubereitungen wird, falls die Gegenwart dieser Substanz erwünscht ist, vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 4,4',4"-(1,3,5-Triazin-2,4,6-triyltriimino)-tris-benzoesäuret-tris(2-ethylhexylester) in den fertigen kosmetischen oder dermatologischen Zubereitungen wird, falls die Gegenwart dieser Substanz erwünscht ist, vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 4-(tert.-Butyl)-4'-methoxydibenzoylmethan in den fertigen kosmetischen oder dermatologischen Zubereitungen wird, falls die Gegenwart dieser Substanz erwünscht ist, vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 4-Methylbenzylidencampher in den fertigen kosmetischen oder dermatologischen Zubereitungen wird, falls die Gegenwart dieser Substanz erwünscht ist, vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 2-Ethylhexyl-p-methoxy-cinnamat in den fertigen kosmetischen oder dermatologischen Zubereitungen wird, falls die Gegenwart dieser Substanz erwünscht ist, vorteilhaft aus dem Bereich von 0,1 - 15,0 Gew.-%, bevorzugt 0,5 - 7,5 Gew.-% gewählt, bezogen auf das Gesamtgewicht der Zubereitungen.

Noch eine weiterere erfindungsgemäß vorteilhaft zu verwendende zusätzliche Lichtschutzfiltersubstanz ist das Ethylhexyl-2-cyano-3,3-diphenylacrylat (Octocrylen), welches von BASF unter der Bezeichnung UVINUL® N 539 erhältlich ist und sich durch folgende Struktur auszeichnet:

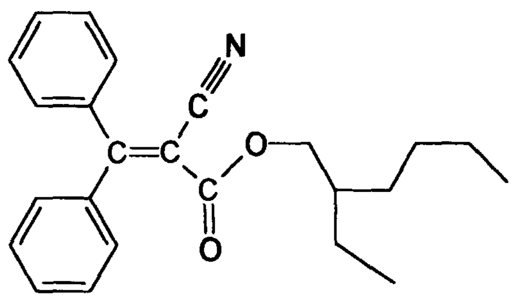

Die Gesamtmenge an Ethylhexyl-2-cyano-3,3-diphenylacrylat in den fertigen kosmetischen oder dermatologischen Zubereitungen wird, falls die Gegenwart dieser Substanz erwünscht ist, vorteilhaft aus dem Bereich von 0,1 - 15,0 Gew.-%, bevorzugt 0,5 - 10,0 Gew.-% gewählt, bezogen auf das Gesamtgewicht der Zubereitungen.

Ferner kann gegebenenfalls von Vorteil sein, erfindungsgemäß weiteren UVA- und/oder UVB-Filtern in kosmetische oder dermatologisch Zubereitungen einzuarbeiten, beispielsweise bestimmten Salicylsäurederivaten wie 4-Isopropylbenzylsalicylat, 2-Ethylhexylsalicylat (=Octylsalicylat), Homomenthylsalicylat.

Die Gesamtmenge an einem oder mehreren Salicylsäurederivaten in den fertigen kosmetischen oder dermatologischen Zubereitungen wird vorteilhaft aus dem Bereich von 0,1 - 15,0 Gew.-%, bevorzugt 0,5 - 8,0 Gew.-% gewählt, bezogen auf das Gesamtgewicht der Zubereitungen. Wenn Ethylhexylsalicylat gewählt wird, ist es von Vorteil, dessen Gesamtmenge aus dem Bereich von 0,1 - 5,0 Gew.-%, bevorzugt 0,5 - 2,5 Gew.-% zu wählen. Wenn Homomenthylsalicylat gewährt wird, ist es von Vorteil, dessen Gesamtmenge aus dem Bereich von 0,1 - 10,0 Gew.-%, bevorzugt 0,5 - 5,0 Gew.-% zu wählen.

Der LSF kosmetischer oder dermatologischer Zubereitungen, enthaltend beiliebige der vorab geschilderten UV-Filtersubstanzen, sei es als Einzelsubstanzen oder in beliebigen Gemischen untereinander, wobei die Gesamtmenge solcher Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen, wird erfindungsgemäß durch die Verwendung von Polymerfiltern erheblich gesteigert.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

Die Bezeichnung „Polymerfilter X" bedeutet in den Beispielen eine Substanz, in welcher Y die Gruppe

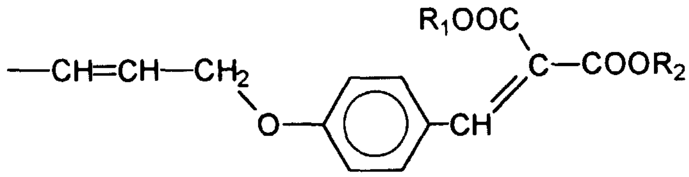

darstellt, in welcher R bzw. R" Methylgruppen darstellen, und in in welchen $R_1$ und $R_2$ Ethylgruppen darstellen und bei welcher das stöchiometrische Verhältnis von Si-O- Einheiten, welche Chromophorgruppen Y tragen, zu Si-O- Einheiten, welche keine Chromophorgruppen Y tragen, etwa 1 : 15 beträgt.

Die Abkürzung BEMPT steht für 2,4-Bis-{[4-(2-Ethyl-hexyloxy)-2-hydroxy]-phenyl}-6-(4-methoxyphenyl)-1,3,5-triazin.

**Beispiel 1**

| O/W-Mikroemulsion | |
|---|---|
| | Gew.-% |
| Ceteareth-12 | 8,00 |
| Cetearylisononanoat | 20,00 |
| Cetearylalkohol | 4,00 |
| Polymerfilter X | 2,00 |
| Eusolex® 232 | 4,80 |
| $MgSO_4$ | 3,00 |
| | pH=5,0 |
| Farbstoffe, Parfum, Konservierungsmittel | q.s. |
| Wasser | ad 100,00 |

Die Bestandteile der Ölphase werden vereinigt und homogenisiert, dann mit der Wasserphase vereinigt und auf eine Temperatur von 80 - 85° C (d.h., in den Phaseninversionstemperaturbereich des Systems) gebracht, hernach auf Raumtemperatur abgekühlt (also aus dem Phaseninversionstemperaturbereich des Systems wieder heraus gebracht).

**Beispiel 2**

| O/W-Mikroemulsion | |
|---|---|
| | Gew.-% |
| Ceteareth-12 | 12,00 |
| Cetearylisononanoat | 20,00 |
| Cetearylalkohol | 6,00 |
| Polymerfilter X | 6,00 |
| Octocrylen | 3,00 |
| Uvinul® T 150 | 2,00 |
| Eusolex® 232 | 4,80 |
| Parsol® 1789 | 2,00 |
| $MgSO_4$ | 3,00 |
| | pH=5,0 |
| Farbstoffe, Parfum, Konservierungsmittel | q.s. |
| Wasser | ad 100,00 |

Die Bestandteile der Ölphase werden vereinigt und homogenisiert, dann mit der Wasserphase vereinigt und auf eine Temperatur von 80 - 85° C (d.h., in den Phaseninversionstemperaturbereich des Systems) gebracht, hernach auf Raumtemperatur abgekühlt (also aus dem Phaseninversionstemperaturbereich des Systems wieder heraus gebracht).

**Beispiel 3**

| O/W-Mikroemulsion | |
|---|---|
| | Gew.-% |
| Ceteareth-12 | 8,00 |
| Cetearylisononanoat | 10,00 |
| Mineralöl | 10,00 |
| Cetearylalkohol | 4,00 |
| Polymerfilter X | 2,00 |
| Uvinul® T150 | 4,80 |
| $MgSO_4$ | 3,00 |
| Farbstoffe, Parfum, Konservierungsmittel | q.s. |
| Wasser | ad 100,00 |

Die unlösliche UV-Filtersubstanz wird im Mineralöl vorsuspendiert und mit der restlichen Ölphase vereinigt, worauf diese und homogenisiert und dann mit der Wasserphase vereinigt und auf eine Temperatur von 80 - 85° C (d.h., in den Phaseninversionstemperaturbereich des Systems) gebracht, hernach auf Raumtemperatur abgekühlt (also aus dem Phaseninversionstemperaturbereich des Systems wieder heraus gebracht) wird.

**Beispiel 4**

| O/W-Mikroemulsion | |
|---|---|
| | Gew.-% |
| Ceteareth-12 | 12,00 |
| Cetearylisononanoat | 10,00 |
| Mineralöl | 10,00 |
| Cetearylalkohol | 6,00 |
| Polymerfilter X | 2,00 |
| BEMPT | 2,00 |
| Eusolex® 6300 | 2,00 |
| Parsol® 1789 | 0,50 |
| Uvinul® T150 | 4,80 |
| $MgSO_4$ | 3,00 |
| Farbstoffe, Parfum, Konservierungsmittel | q.s. |

(fortgesetzt)

| O/W-Mikroemulsion | |
|---|---|
| | Gew.-% |
| Wasser | ad 100,00 |

Die unlösliche UV-Filtersubstanz wird im Mineralöl vorsuspendiert und mit der restlichen Ölphase vereinigt, worauf diese und homogenisiert und dann mit der Wasserphase vereinigt und auf eine Temperatur von 80 - 85° C (d.h., in den Phaseninversionstemperaturbereich des Systems) gebracht, hernach auf Raumtemperatur abgekühlt (also aus dem Phaseninversionsternperaturbereich des Systems wieder heraus gebracht) wird.

**Beispiel 5**

| O/W-Mikroemulsion | |
|---|---|
| | Gew.-% |
| Ceteareth-12 | 8,00 |
| Cetearylisononanoat | 10,00 |
| Cetearylalkohol | 4,00 |
| Polymerfilter X | 1,00 |
| Eusole® 232 | 5,00 |
| Uvinul® T150 | 1,00 |
| $MgSO_4$ | 3,00 |
| | pH=5,0 |
| Farbstoffe, Parfum, Konservierungsmittel | q.s. |
| Wasser | ad 100,00 |

Die Bestandteile der Ölphase werden vereinigt und homogenisiert, dann mit der Wasserphase vereinigt und auf eine Temperatur von 80 - 85° C (d.h., in den Phaseninversionstemperaturbereich des Systems) gebracht, hernach auf Raumtemperatur abgekühlt (also aus dem Phaseninversionstemperaturbereich des Systems wieder heraus gebracht).

**Beispiel 6**

| O/W-Mikroemulsion | |
|---|---|
| | Gew.-% |
| Ceteareth-12 | 8,00 |
| Cetearylisononanoat | 10,00 |
| Polymerfilter X | 10,00 |
| Eusolex® 32 | 2,00 |
| Mineralöl | 10,00 |

(fortgesetzt)

| O/W-Mikroemulsion | |
|---|---|
| | Gew.-% |
| Cetearylalkohol | 4,00 |
| Uvinul® T150 | 5,00 |
| $MgSO_4$ | 3,00 |
| Farbstoffe, Parfum, Konservierungsmittel | q.s. |
| Wasser | ad 100,00 |

Die unlösliche UV-Filtersubstanz wird im Mineralöl vorsuspendiert und mit der restlichen Ölphase vereinigt, worauf diese und homogenisiert und dann mit der Wasserphase vereinigt und auf eine Temperatur von 80 - 85° C (d.h., in den Phaseninversionstemperaturbereich des Systems) gebracht, hernach auf Raumtemperatur abgekühlt (also aus dem Phaseninversionstemperaturbereich des Systems wieder heraus gebracht) wird.

**Beispiel 7**

| O/W/O-Emulsion | |
|---|---|
| | Gew.-% |
| Glycerylisostearat | 5,00 |
| Mineralöl | 25,00 |
| Stearinsäure | 2,00 |
| Polymerfilter X | 3,00 |
| Uvinul® T150 | 2,00 |
| NaOH | ad pH 7,0 |
| Farbstoffe, Parfum, Konservierungsmittel | q.s. |
| Wasser | ad 100,00 |

Die unlösliche UV-Filtersubstanz wird in der Ölphase dispergiert. Wasser wird zugegeben und das System auf ca. 40° C erwärmt. NaOH wird zugegeben bis ein pH-Wert von 7 erreicht ist, hernach wird auf Raumtemperatur abgekühlt.

**Beispiel 8**

| O/W/O-Emulsion | |
|---|---|
| | Gew.-% |
| Polyglyceryl-2-polyhydroxystearat | 7,00 |
| Cetearylisononaoat | 12,50 |
| Mineralöl | 12,50 |

(fortgesetzt)

| O/W/O-Emulsion | |
|---|---|
| | Gew.-% |
| Stearinsäure | 2,00 |
| Polymerfilter X | 5,00 |
| Uvinul® T150 | 2,00 |
| Parsol® 1789 | 2,00 |
| Eusolex® 232 | 2,00 |
| NaOH | ad pH 7,0 |
| Farbstoffe, Parfum, Konservierungsmittel | q.s. |
| Wasser | ad 100,00 |

Die unlösliche UV-Filtersubstanz wird im Mineralöl vorsuspendiert und mit der restlichen Ölphase vereinigt. Wasser wird zugegeben und das System auf ca. 40° C erwärmt. NaOH wird zugegeben bis ein pH-Wert von 7 erreicht ist, hernach wird auf Raumtemperatur abgekühlt.

**Beispiel 9**

| O/W/O-Emulsion | |
|---|---|
| | Gew.-% |
| Polyglyceryl-2-polyhydroxystearat | 5,00 |
| Cetearylisononaoat | 12,50 |
| Mineralöl | 12,50 |
| Stearinsäure | 2,00 |
| Polymerfilter X | 3,00 |
| Eusolex® 232 | 2,00 |
| Uvinul® T150 | 2,00 |
| Eusolex® 6300 | 2,00 |
| Parsol® 1789 | 1,00 |
| NaOH | ad pH 7,0 |
| Farbstoffe, Parfum, Konservierungsmittel | q.s. |
| Wasser | ad 100,00 |

Die unlösliche UV-Filtersubstanz wird in der Ölphase dispergiert. Wasser wird zugegeben und das System auf ca. 40° C erwärmt. NaOH wird zugegeben bis ein pH-Wert von 7 erreicht ist, hernach wird auf Raumtemperatur abgekühlt.

**Beispiel 10**

| O/W-Makroemulsion | |
|---|---|
| | Gew.-% |
| Polyglyceryl-2-polyhydrorystearat | 2,00 |
| Cetearylisononaoat | 12,50 |
| Mineralöl | 12,50 |
| Stearinsäure | 2,00 |
| Polymerfilter X | 7,50 |
| Uvinul® T150 | 2,00 |
| NaOH | ad pH 7,0 |
| Farbstoffe, Parfum, Konservierungsmittel | q.s. |
| Wasser | ad 100,00 |

Die unlösliche UV-Filtersubstanz wird im Mineralöl vorsuspendiert und mit der restlichen Ölphase vereinigt. Wasser wird zugegeben und das System auf ca. 40° C erwärmt. NaOH wird zugegeben bis ein pH-Wert von 7 erreicht ist, hernach wird auf Raumtemperatur abgekühlt.

**Patentansprüche**

1. Lichtschutzwirksame Wirkstoffkombinationen aus unter Normalbedingungen als Festkörper vorliegenden organischen UV-Filterkomponenten und oligomeren oder polymeren UV-Filtersubstanzen mit periodisch sich wiederholenden Si-O- Gruppen.

2. Wirkstoffkombinationen nach Anspruch 1, dadurch gekennzeichnet, daß die oligomeren oder polymeren UV-FIlter-substanzen mit periodisch sich wiederholenden Si-O-Gruppen gewählt werden aus der Gruppe der Substanzen, die durch folgende allgemeine Strukturen beschrieben werden:

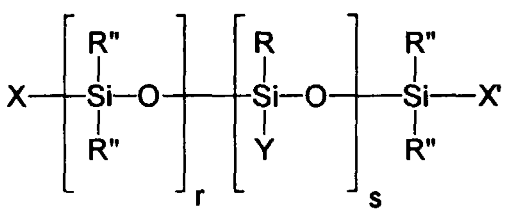

(Strukturschema I)

bzw.

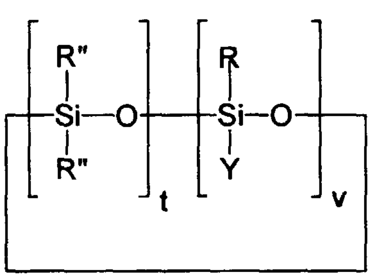

(Strukturschema II),

wobei

- die Reste R und R" Alkylreste mit 1 - 10 Kohlenstoffatomen bzw. Phenylreste repräsentieren.
- die Reste X, X' entweder ebenfalls organische Reste wie R und R" oder aber Reste wie Y, repräsentieren
- r Werte von 0 - 100 annimmt
- s Werte von 0 - 20 annimmt, wobei mindestens einer der Reste X, X' einen Rest Y bedeutet, wenn s = 0.
- t Werte von 0 - 10 annimmt,
- v Werte von 1 - 10 annimmt, wobei die Summe aus v und t größer oder gleich 3 ist.
- Y stellt die für die UV-Absorption verantwortlichen Chromophorreste dar und insbesondere vorteilhaft gewählt wird aus der Gruppe

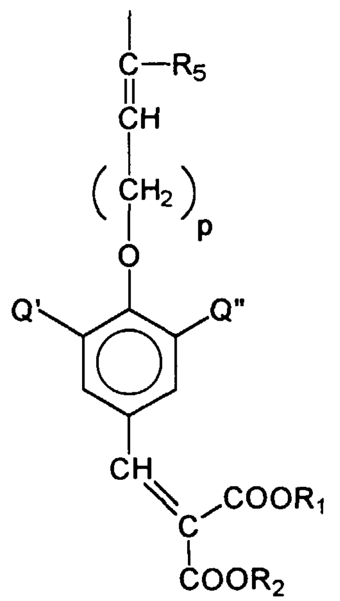

**und/oder**

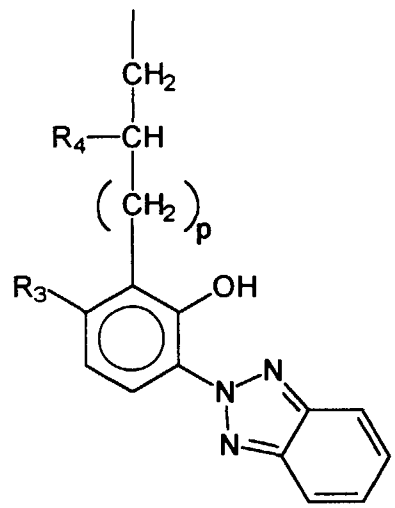

,

wobei $R_1$, $R_2$, unabhängig voneinander verzweigte oder unverzweigte Alkylgruppen von 1 - 10 Kohlenstoffatomen darstellen, und wobei $R_3$, $R_4$, $R_5$ unabhängig voneinander Wasserstoffatome bzw. verzweigte oder unverzweigte Alkylgruppen von 1 - 10 Kohlenstoffatomen darstellen und p eine Zahl von 1 - 10 darstellt. Q' und/oder Q" können unabhängig voneinander darstellen: H, Hydroxygruppen, verzweigte oder unverzweigte Alkylgruppen von 1 - 10 Kohlenstoffatomen, verzweigte oder unverzweigte Alkoxygruppen mit 1 - 10 Kohlenstoffatomen.

**3.** Wirkstoffkombinationen nach Anspruch 1 , dadurch gekennzeichnet, daß die unter Normalbedingungen als Festkörper vorliegenden organischen UV-Filterkomponenten gewährt werden aus der Gruppe

- 4,4',4"-(1,3,5-Triazin-2,4,6-triyltriimino)-tris-benzoesäure-tris(2-ethylhexylester)
- 2,4-Bis-{[4-(2-Ethyl-hexyloxy)-2-hydroxy]-phenyl}-6-(4-methoxyphenyl)-1,3,5-triazin,
- 2,4-Bis-{[4-(3-sulfonato)-2-hydroxy-propyloxy)-2-hydroxy]-phenyl}-6-(4-methoxyphenyl)-1,3,5-triazin Natriumsalz,
- 2,4-Bis-{[4-(3-(2-Propyloxy)-2-hydroxy-propyloxy)-2-hydroxy]-phenyl}-6-(4-methoxyphenyl)-1,3,5-triazin,
- 2,4-Bis-{[4-(2-ethyl-hexyloxy)-2-hydroxy]-phenyl}-6-[4-(2-methoxyethyl-carboxyl)-phenylamino]-1,3,5-triazin,
- 2,4-Bis-{[4-(3-(2-propyloxy)-2-hydroxy-propyloxy)-2-hydroxy]-phenyl}-6-[4-(2-ethyl-carboxyl)-phenylamino]-1,3,5-triazin,
- 2,4-Bis-{[4-(2-ethyl-hexyloxy)-2-hydroxy]-phenyl}-6-(1-methyl-pyrrol-2-yl)-1,3,5-triazin,
- 2,4-Bis-{[4-tris(trimethylsiloxy-silylpropyloxy)-2-hydroxy]-phenyl}-6-(4-methoxy-phenyl)-1,3,5-triazin,
- 2,4-Bis-{[4-(2"-methylpropenyloxy)-2-hydroxy]-phenyl}-6-(4-methoxyphenyl)-1,3,5-triazin,
- 2,4-Bis-{[4-(1',1',1',3',5',5',5'-Heptamethylsiloxy-2"-methyl-propyloxy)-2-hydroxy]-phenyl}-6-(4-methoxyphenyl)-1,3,5-triazin,
- 2-Phenylbenzimidazol-5-sulfonsäure bzw. deren Salze, insbesondere das Natrium-,das Kalium und das TEA-Salz.

4. Verwendung von oligomeren oder polymeren UV-FIltersubstanzen mit periodisch sich wiederholenden Si-O- Gruppen zur Erhöhung des Lichtschutzfaktors kosmetischer oder dermatologischer Zubereitungen, welche mindestens eine unter Normalbedingungen als Festkörper vorliegende UV-Filtersubstanz enthalten.

5. Verwendung von oligomeren oder polymeren UV-Filtersubstanzen mit periodisch sich wiederholenden Si-O- Gruppen zur Erzielung, Erhöhung bzw. Verbesserung der Dispergierbarkeit von unter Normalbedingungen als Festkörper vorliegenden UV-Filtersubstanzen in kosmetischen oder pharmazeutischen Zubereitungen.